Europäisches Patentamt

European Patent Office

Office européen des brevets

(19)

(11) Publication number: **0 388 151**

**A1**

(12) **EUROPEAN PATENT APPLICATION**

(21) Application number: 90302668.0

(22) Date of filing: 13.03.90

(51) Int. Cl.5: **C12N 15/13, C12P 21/08, C12N 5/10, G01N 33/577**

(30) Priority: 13.03.89 GB 8905669

(43) Date of publication of application:
19.09.90 Bulletin 90/38

(84) Designated Contracting States:
AT BE CH DE DK ES FR GB GR IT LI LU NL SE

(71) Applicant: CELLTECH LIMITED
216 Bath Road
Slough Berkshire SL1 4EN(GB)

(72) Inventor: Adair, John Robert
23 George Road
Stokenchurch, High Wycombe HP14 3RN(GB)
Inventor: Yarranton, Geoffrey Thomas
8 Sherwood Road, Winnersh.
Nr. Wokingham, Berkshire RG11 7NJ(GB)
Inventor: King, David John
59 Yaverland Drive
Bagshot, Surrey GU1 5DY(GB)

(74) Representative: Mercer, Christopher Paul et al
Carpmaels & Ransford 43, Bloomsbury Square
London WC1A 2RA(GB)

(54) Modified antibodies.

(57) There is provided a modified antibody having an altered number of glycosylation sites as compared to the number in the equivalent natural antibody. Preferably the modified antibody is produced by recombinant DNA techniques involving the production of an expression vector containing a DNA sequence encoding a modified antibody chain having an altered number of glycosylation sites. The DNA sequence may be prepared from the natural antibody gene by site-directed mutagenesis. Preferably the modified antibody chain has an increased number of glycosylation sites; for instance a modified Fab, Fab' or (Fab')$_2$ antibody with a glycosylation site introduced into the CL or CH1 domain thereof. Altering the degree of glycosylation alters properties of the antibodies, e.g. blood clearance rate, when used in vivo; generally the greater the degree of glycosylation the slower the blood clearance. The altered antibodies may be used for the preparation of compositions for use in diagnosis, e.g. in vivo diagnosis and imaging, or in therapy; glycosylation minus/reduced antibodies being preferred for diagnostic uses and glycosylation plus/increased antibodies being preferred for therapeutic application.

# MODIFIED ANTIBODIES

Field of the Invention

The present invention relates to modified antibodies, processes for their production by recombinant DNA technology, pharmaceutical and diagnostic compositions comprising the modified antibodies, and the use of the modified antibodies or compositions in immunoassays, and in vivo imaging or therapy.

In the description, reference is made to a number of publications by number. The publications are listed in numerical order at the end of the description.

Background to the Invention

Natural immunoglobulins (Igs) have been known for many years, as have the various fragments thereof, such as the Fab, (Fab')₂ and Fc fragments, which can be derived by enzymatic cleavage. Natural Igs comprise a generally Y-shaped molecule having an antigen binding site at the end of each arm. The remainder of the structure, and particularly the stem of the Y, mediates the effector functions associated with Igs.

In this specification the abbreviation "Ig" is used to refer to the complete Y-shaped Ig molecule, whereas "antibody", alone or in combination, is used to refer to both Igs and their fragments, provided that the fragment contains at least one antigen binding site.

Natural Igs have been used in diagnosis and, to a more limited extent, in therapy. However, such uses, especially in therapy, have been hindered by the polyclonal nature of natural Igs. A significant step towards the realisation of the potential of Igs as therapeutic agents was the discovery of techniques for the preparation of monoclonal antibodies (MAbs) [1] of defined antigen specificity. Most MAbs are produced by hybrid cells prepared by fusion of rodent spleen cells with rodent myeloma cells.

It has been widely suggested that Igs, and in particular MAbs, potentially could be very useful in the diagnosis and treatment of cancer [2, 3]. There has therefore been much activity in trying to produce Igs or MAbs directed against tumour-specific antigens. So far, over one hundred MAbs directed against a variety of human carcinomas have been used in various aspects of tumour diagnosis or treatment [4].

Natural Igs and MAbs are generally glycosylated, that is they have carbohydrate moieties attached to their polypeptide chains. The carbohydrate moieties are typically attached at specific amino acid residues located within particular structural features of the polypeptide chains. Thus, for example, carbohydrate moieties can be N-linked to asparagine residues by a series of enzyme-catalysed reactions. For carbohydrate attachment these asparagine residues are typically located in a loop or β-turn and in a sequence Asn-X-Y wherein R can be any amino-acid apart from Pro, Trp or Asp and Y is Ser or Thr.

In the constant regions of human IgGs there is a single site suitable for N-linked glycosylation in the CH2 domain of each heavy chain at asparagine residue 314 (using the residue numbering set forth by Kabat et al [5]). Therefore each assembled IgG has two N-linked carbohydrate moieties. These are located in relatively close association on the inner surface of the Fc region within an area of major contact between the heavy chain CH2 domains.

It is well known that tunicamycin can inhibit the glycosylation of proteins. Nose and Wigzell [6] have investigated the significance of glycosylation of antibodies with MAbs produced by hybridomas grown in tunicamycin-containing medium. They found that the absence of carbohydrate did not appear to affect the fine antigen binding reactivity or protein A binding capacity of the MAb.

However, unglycosylated MAbs lost the ability to activate complement, to bind to Fc receptors on macrophages and to induce antibody-dependent cellular toxicity. They also examined the effect of glycosylation on the elimination of antigen-antibody complexes from the circulation. It was found that the rate of elimination of unglycosylated antibody-antigen complexes was less than that for glycosylated antibody-antigen complexes and about the same as the rate for antigen alone. However, unglycosylated antibody by itself was eliminated at about the same rate as glycosylated antibody.

The use of tunicamycin to inhibit glycosylation has several adverse effects on cellular metabolism, and thus employing tunicamycin to produce useful quantities of unglycosylated antibodies is not a practical proposition.

Morrison et al (Morrison, S.L., Canfield, S., Porter, S., Tan, L.K., Tao, M., and Wims, L.A.; Clinical Chemistry, Vol. 34, No. 9, 1988, pages 1668-1675) have reported that the use of site-directed mutagenesis to remove the carbohydrate addition signal from the CH2 domain of Igs. However, no detailed description of the experiments which were carried out or of the results which were obtained were given. There is no indication that unglycosylated antibody molecules were obtained, nor that such alterations gave rise to modification of biological function.

## Summary of the Invention

According to a first aspect of the present invention, there is provided a process for the production of an antibody having an altered number of glycosylation sites which comprises producing an expression vector containing a DNA sequence encoding a modified antibody chain having a number of glycosylation sites which is different from the number of glycosylation sites present in the natural chain from which the modified chain is derived.

The antibody chain may be a complete heavy or light chain or a fragment thereof, such as a truncated heavy chain comprising the VH and CH1 domains or VH, CH1 and hinge domains. The antibody chain may comprise a VH or VL fragment.

The modified chain may have a reduced number of glycosylation sites. This may be achieved by altering the DNA sequence encoding one, some or all of the amino acid residues required for a glycosylation site present in a natural chain. Thus, for example, the DNA corresponding to either the Asn or the Thr/Ser residue of the natural glycosylation site may be altered, e.g. at residues 314 or 316 of the human IgG CH2 domain. In addition to the DNA sequences which encode the amino acid residues which are at the natural glycosylation site DNA encoding nearby and other amino acid residues required for the carbohydrate binding function of the natural glycosylation site may be altered also.

Preferably the modified chain has an increased number of glycosylation sites. For instance, natural human Fab, Fab' or (Fab')₂ fragments do not normally contain any glycosylation sites. A glycosylation site may be introduced in any of the domains of such fragments. Preferably, a glycosylation site is introduced into the CL or CH1 domain of a natural antibody sequence used to produce a modified Fab or (Fab')₂ antibody. This may be achieved by selecting a suitable loop or β-turn area and altering the DNA sequence encoding this area so that it encodes a glycosylation site.

Preferably, the sequence alteration(s) in the DNA, to introduce or remove glycosylation sites, is (are) carried out by site directed mutagenesis. Polymerase Chain Reaction (PCR) protocols may be used. However, oligonucleotide synthesis techniques may also be used to synthesise DNA sequences containing the desired sequence alterations. Such synthesised sequences may comprise the whole or part of the DNA sequence which encodes the complete antibody chain.

Preferably, the method of this aspect of the invention includes the steps of:
(b) transfecting or transforming host cells with the vector; and
(c) culturing the transformed host cells to produce an antibody molecule having an altered number of glycosylation sites.

If desired, the antibody which is to be altered may be a "chimeric" or "humanised" antibody produced by processes such as those described in WO 86/01533 or EP-A-0239400.

In the process of this aspect of the present invention, if the vector encodes only a single antibody polypeptide chain, the product of the process may be a dimeric molecule. If a tetrameric molecule similar to a natural Ig or (Fab')₂ fragment or a dimeric molecule similar to an Fab fragment is required, various alternative strategies may be employed.

In the first alternative, the host cells may also be transfected or transformed with a second vector which encodes a complementary antibody heavy or light chain. Preferably, the vectors are such as to ensure as far as possible that each polypeptide chain is expressed at substantially similar levels. For example, vectors which are identical except in so far as the coding sequence of the antibody and selectable markers are concerned may be used.

In the second alternative, the first vector may include DNA sequences coding for complementary light chain- and heavy chain-derived polypeptides.

In a further alternative, if the vector encodes only a heavy chain (or light chain) polypeptide, it is also possible to produce a recombinant antibody molecule by using a host cell which naturally secretes a complementary light chain (or heavy chain).

Where the second antibody chain is produced by expression from a vector, the sequence encoding the complementary antibody chain may also be modified to alter the number of glycosylation sites.

Where the modified antibody includes at least one glycosylation site and it is desired to produce a glycosylated product, the host cell will need to be a eucaryotic, preferably a mammalian, host cell. Such host cells, in contrast to prokaryotic host cells, have the necessary cellular machinery to glycosylate the modified antibody. preferred host cells are Chinese Hamster Ovary (CHO) cells and myeloma or hybridoma cells. Yeast host cells, e.g. Saccharomyces cerevisiae host cells, may also be used to produce the modified antibodies. If the modified antibody has no glycosylation sites, or it is desired to produce a non-glycosylated product, the host cell can also be a prokaryotic cell, e.g. E. coli.

By use of the process of the first aspect of the present invention, not only is it possible to produce unglycosylated antibodies without the need to use tunicamycin, but also it is possible to produce "unnaturally" glycosylated antibodies, i.e. antibodies glycosylated at sites at which they are not normally glycosylated as well as glycosylated antibodies in which the attached carbohydrate is different from that normally found in natural glycosylated Igs.

Therefore, according to the second aspect of the present invention, there is provided a modified antibody and in particular an Fab or (Fab')$_2$ Ig fragment, having an altered number of glycosylation sites as compared to the number in the equivalent natural antibody.

The glycosylation sites of the antibodies of the second aspect of the invention may have carbohydrate attached thereto or may be vacant. Preferably these antibodies have carbohydrate attached for end use e.g. for therapeutic or diagnostic use.

According to a third aspect of the present invention, there is provided a composition comprising a modified antibody having a number of glycosylation sites which is altered compared to the number of such sites in the equivalent natural antibody and a pharmaceutically-or diagnostically-acceptable carrier, excipient or diluent.

The modified antibodies of the present invention may be any antibody for which it is desired to add or subtract glycosylation sites. Thus the antibody may comprise a site or tissue specific antibody such as an antibody having fibrin specificity or specificity for a tumour associated antigen. Alternatively the antibody may have specificity for a biologically functional molecule such as a lymphokine, e.g. interferon $\gamma$, TNF, or IL2, or a receptor e.g. cell surface receptor, for such a biologically functional molecule, or cell surface antigen, e.g. a T cell or leucocyte cell surface antigen.

Preferably, the modified antibody is made by the process according to the first aspect of the present invention.

If desired, an effector or reporter molecule may be attached to the modified antibody. For diagnostic applications, the reporter molecule may be an enzyme, a radiolabel, a fluorescent label, a ligand molecule (e.g. biotin or avidin) or any of the other labels commonly used in immunoassays or for imaging. Where the modified antibody has an increased number of glycosylation sites, the carbohydrate linked to the glycosylation site may be used by itself as a label or may be used for attaching a label or effector molecule to the antibody. Suitable effector molecules include enzymes, such as plasminogen activator enzymes, toxins, such as ricin or its fragments, and radiotherapy agents.

The modified antibodies of the present invention may be used for therapy or for in vitro or in vivo diagnosis. The alteration of the degree of glycosylation of the antibody allows more control to be exercised over the time course of an in vivo diagnostic or therapeutic procedure. In accordance with the invention we have found that abolishing or decreasing glycosylation increases the rate at which the antibody is cleared from the blood in vivo; whereas adding, or increasing the degree of glycosylation gives rise to slower blood clearance. Thus, advantageously, glycosylation minus/decreased antibodies may be more suitable for use in in vivo diagnostic and imaging applications and glycosylation plus/increased antibodies may be more suitable for use in therapeutic applications.

The present invention also includes cloning and expression vectors and transformed host cells used in the process of the first aspect of the present invention as well as the diagnostic and therapeutic uses of the modified antibodies.

The general methods by which the vectors may be constructed, transfection methods and culture methods are well known per se and form no part of the invention. Such methods are shown, for instance in references [7] and [8].

The present invention is now described, by way of illustration only in the following examples which refer to the accompanying drawings in which:

Brief Description of the Drawings

Figure 1 shows the construction of a vector containing a DNA sequence encoding a modified heavy chain;

Figure 2 shows analysis of COS cell expression products using SDS-PAGE;

Figure 3 shows analysis of COS cell expression products using antigen binding assays;

Figure 4 shows the results of tissue uptake experiments using labelled antibodies;
Figure 5 shows analysis of COS cell expression products using reducing PAGE, and
Figure 6 shows analysis of CHO cell expression products using SDS-PAGE.

Description of Specific Embodiments of the Invention

Example 1

Deletion of natural glycosylation recognition site in chimeric B72.3 protein and expression of the altered protein in COS and CHO cells

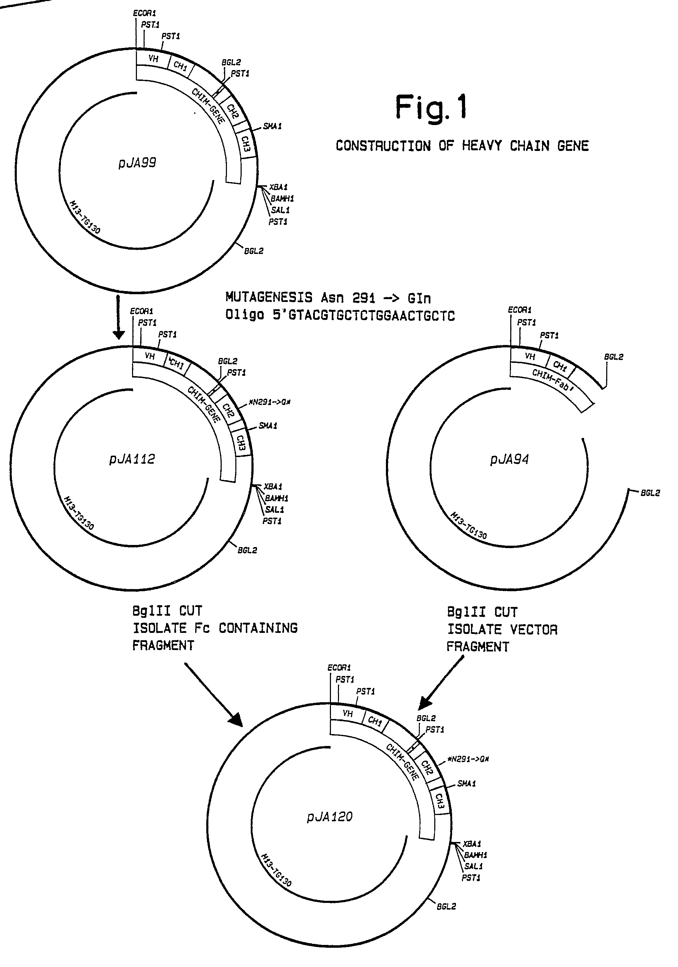

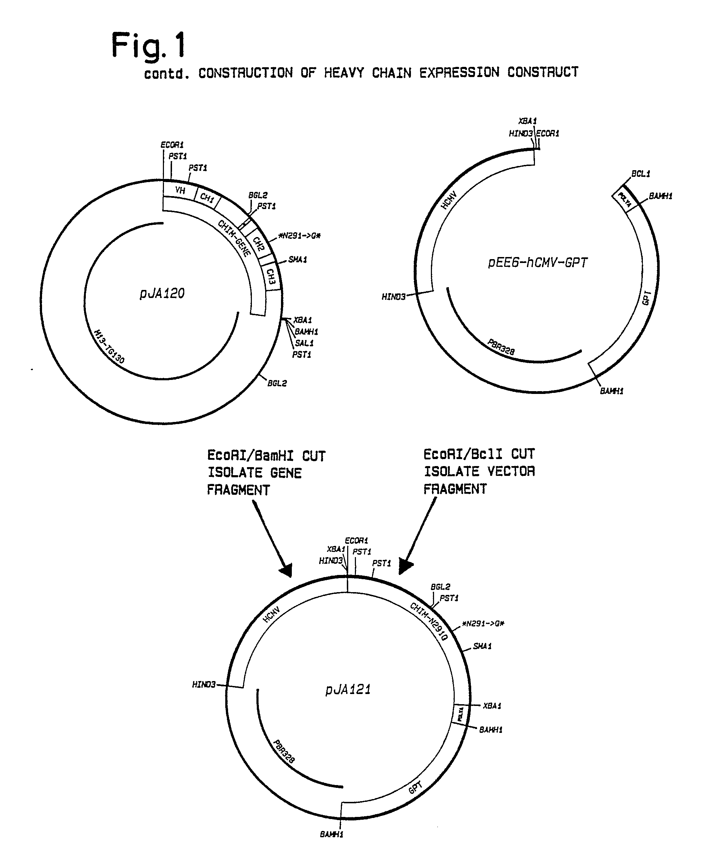

A vector containing an Ig heavy chain gene which had been modified to alter the sequence of the natural chain's single glycosylation site was constructed. The construction of the vector is shown in Figure 1 to which reference is now made.

Plasmid JA99 contains a mouse/human chimeric heavy chain gene [9]. This chimeric protein is also described in published International Patent Application WO 89/01782 and is derived from a mouse MAb B72.3. Details of the B72.3 MAb and its humanised derivatives are given in published International patent Application WO 89/01783.

The chimeric heavy chain gene was cloned via its unique EcoRI and BamHI sites into bacteriophage M13tg130 [10]. This heavy chain gene has a single glycosylation site in the CH2 domain beginning with an Asn residue at residue 291 of the linear amino acid sequence (position 314 according to the numbering of kabat et al, 1987). Single stranded plasmid DNA was prepared as described by Zinder and Boeke [11]. An oligonucleotide of the following sequence:

5′ GTA CGT GTC CTG GAA CTG CTC (21 mer)

was prepared. This oligonucleotide was designed to enable the sequence at the glycosylation site in the CH2 domain to be altered by site directed mutagenesis by replacing the codon encoding Asn 291 with a codon encoding Gln. Mutagenesis was carried out as described by Zoller and Smith [12].

Mutant phage were identified by stringent hybridisation with the above oligonucleotide. Candidate mutants were picked, single stranded DNA was prepared and DNA sequences were derived using a set of sequencing primers spaced throughout the Fc region of the constant gene. One clone, JA112, was taken and double stranded DNA was prepared as described by Zinder and Boeke [11]. A 2.6 kbp BglII fragment containing the Fc region of the constant region gene was isolated and ligated to a 7.7 kbp BglII fragment from plasmid JA94, an M13 vector containing the Fab′ region of the chimeric gene. In this way the chimeric heavy chain gene was reconstructed without the need to sequence all of the chimeric gene.

The resultant M13 RF DNA, JA120, was cut with EcoRI and BamHI and the modified gene, encoding a heavy chain lacking a glycosylation site (Δ CHO), was removed as a 2.3 kbp fragment. The 2.3 kbp fragment was ligated to an EcoRI-BclI phosphatased vector fragment of the expression vector EE6-hcmv-gpt [9] to give plasmid JA121. The construction of pJA121, starting from pJA99 is illustrated in Figure 1. pJA121 was used for analysis of the heavy chain in transient expression systems and for the establishment of stable lines in CHO-K1 cells.

The corresponding chimeric mouse/human light chain gene has been isolated and inserted into the expression vector EE6-hCMV-neo as described in Whittle et al [9] and published International Patent Application WO 89/01783. This expression vector is identical to EE6-hCMV-gpt except that the gpt resistance gene has been replaced by a neomycin resistance gene. The resultant EE6-derived light-chain-containing plasmid is called plasmid EE6-cL-neo. This plasmid was transfected into CHO K1 cells and a stable chimeric light chain-producing cell line cL18 established as described in published International Patent Application WO 89/01783.

Transfection of COS cells with the expression vectors pJA121 and EE6-cL-neo was carried out according to the protocol described by Whittle et al [9]. The cells were incubated for 6 hours in a DNA-DEAE dextran solution for six hours and then shocked for two minutes with 10% DMSO in HEPES-buffered saline. The cells were then washed and incubated in medium containing 10% foetal calf serum (FCS) for 72 hours. The medium was removed for analysis and the cells were lysed using 500 μl of 1% Triton X-100, 0.5% deoxycholate, 0.1% SDS, 0.01M sodium phosphate, 0.1M NaCl and 0.001M EDTA, pH 7.5 per $10^5$ cells. Lysates and conditioned media were centrifuged for five minutes in an Eppendorf centrifuge to remove nuclei and cell debris and stored at 4°C before analysis.

Microtitre plates were coated with 0.25 μg per well of sheep or goat antibody reactive against human epitopes on the heavy or light chains (Jackson Immuno Research, Serotec; Cappel; Tago). Supernatants or lysates from transfected COS cells were diluted 1:2 or 1:4 respectively in sample conjugate buffer containing 0.1M Tris-HCl, 0.1M sodium chloride, 0.02% Tween 20 and 0.2% casein, pH7.0. 100 $\mu$l of each diluted sample were added to each well and incubated for 1 hour at room temperature with gentle agitation. Following washing six times with wash buffer (phosphate buffered saline containing 0.2% Tween 20, pH 7.2), 100 $\mu$l of a 1:5000 dilution of standard horseradish peroxidase (HRP)-conjugated antibody reactive against either human or mouse specific epitopes were added per well. The plates were incubated for 1 hour at room temperature, and then washed six times with wash buffer. 100 $\mu$l of substrate buffer containing 0.1 mg/ml tetramethylbenzidine (TMB), 0.1M sodium citrate, pH 6.0 and 0.005% $H_2O_2$ were added to each well to generate a colour change. The reaction was terminated after 2-3 minutes by adjusting the solution to pH 1 with 1.5M sulphuric acid. The optical density was determined at 450 nm for each well by measurement in a Dynatech laboratories MR600 microplate reader. Standard curves were generated using known concentrations of appropriate immunoglobulins.

Antigen binding assays were performed in an analogous manner. Microtitre plates were coated with 0.25 $\mu$g per well of TAG-72 antigen, purified according to the method of Johnson et al [13], obtained from tumour xenografts implanted in nude mice. (The TAG-72 antigen is recognised by the mouse MAb B72.3 from which the heavy and light chain genes described above are derived).

Following washing six times in wash buffer, samples from COS cell transfections were added as previously, and the same subsequent procedures carried out, using goat anti-human (Fab')$_2$ linked to HRP as the second antibody.

Following transfection, COS cells were allowed to recover in DMEM containing 10% foetal calf serum, for 24 hours. The medium was then replaced with methionine-free DMEM, to which [$^{35}$S] methionine had been added at 200 $\mu$Ci/ml. The cells were metabolically labelled for 48 hours, and conditioned media and cell lysates prepared as previously. Affinity-purified rabbit antibodies against human IgG (Fab')$_2$ and human K chain were used for immunoprecipitations, following coupling to Protein A-Sepharose. Secreted antibodies were analysed on an SDS-10% PAGE system under reducing and non-reducing conditions. Cells were treated with an autoradiography enhancer, dried and exposed to Fuji RX film.

To inhibit N-linked glycosylation chemically, COS cells were grown in medium containing 10 $\mu$g/ml tunicamycin diluted from a stock solution. To ensure that the pool of lipid-linked oligosaccharide was depleted, the cells were maintained in the tunicamycin-containing medium for 2 hours prior to addition of radioactive label.

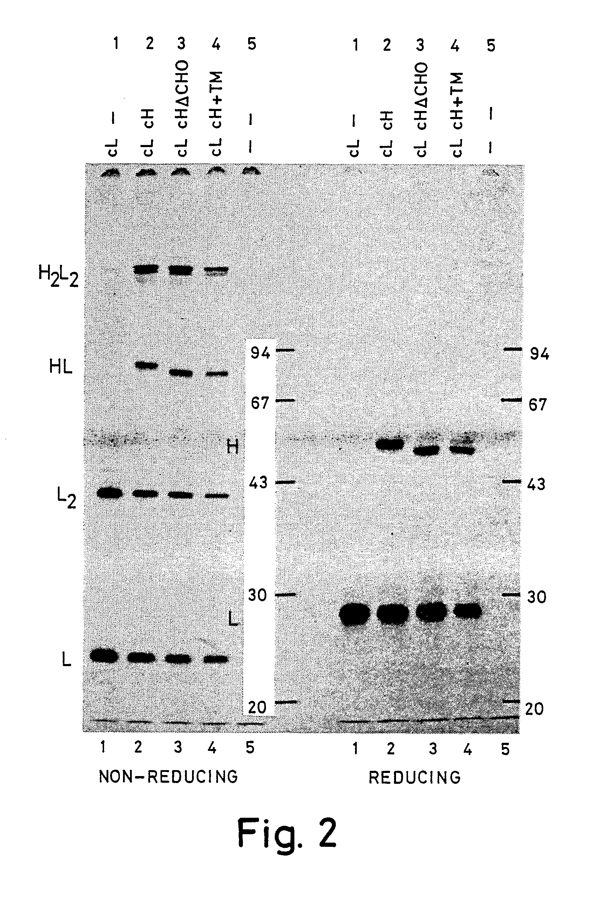

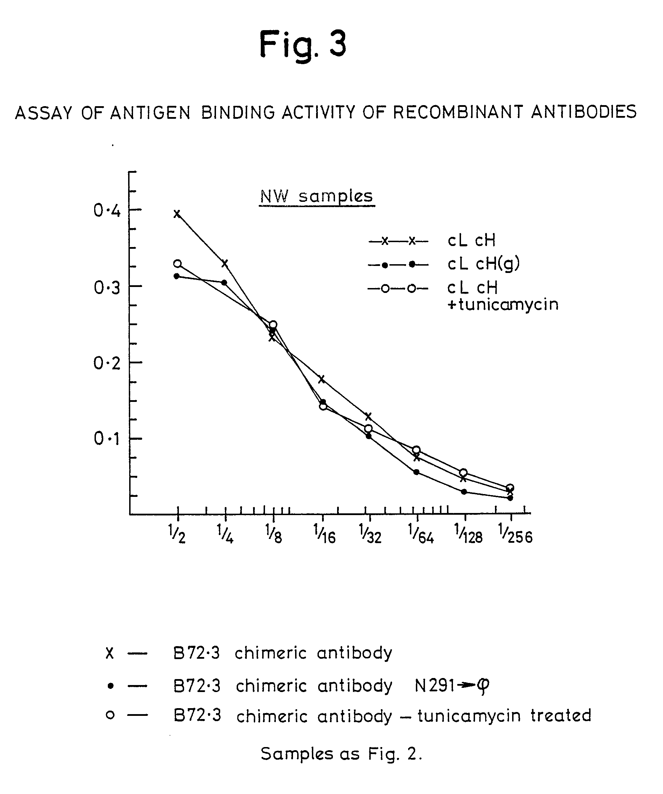

Figure 2 shows the results of the analyses by PAGE of products produced using COS cell expression. In the left hand panel, the products were not reduced, whereas they were in the right hand panel. The centre line shows molecular weight markers. For each panel, the plasmids used for transfection are as follows:

Lane 1 - pEE6-cL-neo (light chain alone);
Lanes 2 and 4 - pEE6-cL-neo and pJA96 (light chain and natural heavy chain) Lane 3 - pEE6-cL-neo and pJA121 (light chain and modified heavy chain); and
Lane 5 control
Lane 4 represents the results of expression in the presence of tunicamycin.

It can be seen from this Figure that when the modified gene or tunicamycin is used, the molecular weight of the heavy chain has been reduced, thus indicating that there is no glycosylation in these cases. The light chain is unaffected.

Figure 3 shows the results of a comparison between the antigen binding activity of the COS cell expression products corresponding to Lanes 2 to 4 of Figure 2 above. From this it can be seen that the presence or absence of glycosylation makes no significant difference to antigen binding activity.

CHO cell line cL18 (WO 89/01783) is capable of expressing the light chain gene contained on plasmid pEE6-cL-neo. CL1B cells were grown in attached culture in DMEM medium containing 10% FCS, 2mM glutamine, and non-essential amino acids. Confluent flasks were trypsinized, the cells were washed in phosphate buffered saline (PBS) and were spun down in a bench centrifuge (2000 rpm for 10 min.). Pelleted cells were resuspended in PBS at 1 x $10^7$ cells/ml and stored at 4°C.

Plasmid pJA121 was linearised by cleavage with SalI. 40 $\mu$g of linearised plasmid DNA were added to 1ml of the stored cells ($10^7$ cells) and subjected to electroporation using two successive 2000 V pulses. After 10 minutes at 4°C, growth medium was added and cells were plated out in 90mm petri dishes ($10^6$ cells per plate). After incubation at 37°C for 24 hours, growth medium was removed from the cells and replaced with selective growth medium containing: DMEM, 10% FCS, 2$\mu$M glutamine, non-essential amino acids, 02 mg/ml Xanthine, hypoxanthine and thymidine, 10mg/ml mycophenolic acid and HCl to adjust pH to 7.5. Mycophenolic acid is toxic to cells in this medium, unless the enzyme xanthine-guanosine phosphoribosyltransferase (XGPRT) is present. The gene (gpt) for this enzyme is found on the expression vector pJA121.

After 3 weeks incubation, colonies were picked into 24 well plates, and when growth was semi-confluent, expression of antibody was screened for, by ELISA antigen binding assay [9]. Colonies expressing antibody were expanded until supernatants from roller bottle culture could be supplied for protein purification.

Antibody was purified from cell culture supernatant by affinity chromatography on protein A Sepharose. A column of protein A Sepharose was equilibrated with 50ml glycine/sodium glycinate buffer at pH 8.8 and the supernatant from the cell culture applied directly to the column after adjusting the pH to 8.8 with 0.2M sodium glycinate. After sample loading, the column was washed with equilibration buffer and the antibody eluted by applying a gradient of decreasing pH made up of 0.2M disodium hydrogen phosphate and 0.1M citric acid. The fractions containing antibody were pooled, dialysed into 50mM sodium phosphate buffer pH7.5 and concentrated by ultrafiltration in an Amicon stirred cell using a YM10 membrane.

The purified antibody was shown to be fully assembled by SDS-polyacrylamide gel electrophoresis and was shown to bind the TAG72 antigen in an ELISA. The results of these analyses were similar to those shown in Figures 2 and 3.

Purified mouse MAb B72.3, chimeric mouse/human B72.3 and unglycosylated chimeric B72.3 (produced by the transfected cL18 cells) antibodies were radiolabelled with $^{125}$I by the chloramine-T method [14].

Groups of four male nude mice were given subcutaneous xenografts of tumour LS174T which expresses the TAG-72 antigen recognised by the B72.3 antibodies. Each mouse in each group was injected with 100$\mu$Ci of radiolabelled mouse, mouse/human or unglycosylated antibody in 0.1 ml PBS. Groups of animals were sacrificed at intervals for the collection of tissue samples which were weighed, dissolved in 6M KOH and counted in an LKB Model 1270 "Rackbeta" counter. Tissue uptake was calculated as the mean percentage of injected dose per gram of tissue from a group of four animals.

For imaging, the animals were anaesthetised at intervals and scanned using a Technicare Gemini gamma camera fitted with a pinhole collimator and Saturn computer system.

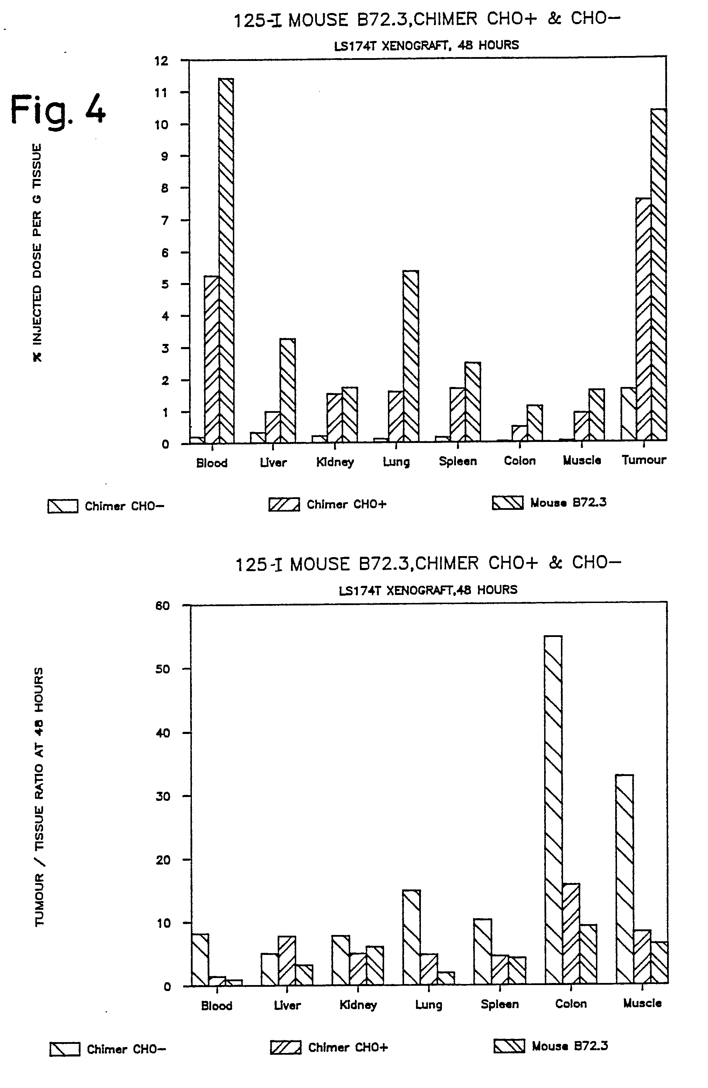

The results of the tissue uptake studies are shown in Figure 4. This shows in the top panel the tissue distribution of the respective antibodies 48 hours after injection. In the bottom panel the tumour/tissue ratio 48 hours after injection is shown. These results show that the unglycosylated chimeric antibody is localised on the tumour but has a lower tumour uptake than the two glycosylated variants. However, the unglycosylated antibody is more rapidly cleared from the blood, giving much higher tumour/blood ratios.

The results of the scans with the gamma camera showed that the unglycosylated antibody produces images which are as easy to interpret as are those given using the glycosylated variants.

It is thus believed that the unglycosylated antibody will be of particular use in imaging applications.


Example 2


Introduction of a recognition site for asparagine-linked (N-linked) glycosylation at location 172 on the CH1 domain of the Fd chain of a chimeric B72.3 F(ab) protein and its expression in COS and CHO cells


Introduction of the required sequence motif for N-linked glycosylation may be achieved by site directed mutagenesis (SDM) or gene synthesis. The preferred route is SDM. Preferably only one amino acid should be altered to minimise the potential for adverse structural effects, however any or all of the three residues in the recognition motif could be substituted into a given location if the tertiary structure requirements at that location were particularly suitable.

Clone mJA108 has been described (WO 89/01783). ssDNA was prepared and used in a SDM experiment using the oligonucleotide

5′G TCC TGA GGA GTT TAG GAC AGC C3′

which can cause the alteration of the sequence Gln-Ser-Ser to Asn-Ser-Ser at residues 172-174 of the chimeric B72.3 sequence when the sense strand of the B72.3 heavy chain gene is cloned in an M13 vector. Mutagenesis was carried out as described by Kramer et al [14]. Screening for mutant clones was as described in Example 1.

A potential mutant, mJA153 was identified and the presence of the desired nucleotide alteration was confirmed by DNA sequencing. dsDNA was prepared and a 1.44kbp region from the Sal1 site at the 3′ end of the CH1 domain to the Bgl11 site in the M13 genome was removed and replaced by a 705 bp Sal1-Bgl11 fragment from mJA107. This fragment includes a synthetic Sal1-EcoR1 sequence which can code for the remainder of the CH1 domain, a synthetic human IgG4 hinge with an altered cysteine pattern such that the second cysteine of the IgG4 hinge is changed to an alanine, and translation stop codons at the end of the hinge coding sequence. Use of this sequence to form a functional Fd fragment has been described in PCT/GB 88/00731.

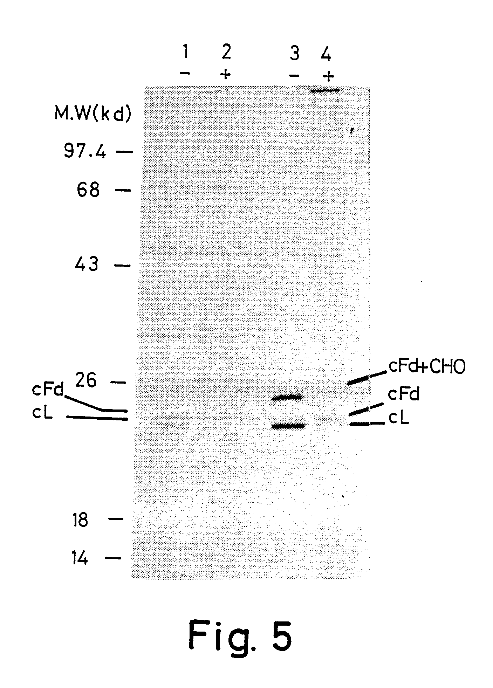

The resultant vector contains an 760 bp EcoR1 fragment which can code for a functional B72.3 chimeric Fd chain. This fragment was removed and cloned into the unique EcoR1 site of EE6.hCMV.gpt (WO 89/01783) to give pEE6cFd.δcys.Carb. DNA was prepared and used to transfect COS-7 cells in association with the chimeric light chain plasmid EE6.cL.neo, in the presence or absence of tunicamycin to demonstrate that glycosylation of the Fd chain occurs in vivo. Procedures for the use of tunicamycin are as for Example 1. $^{35}$S labelled protein was produced from the COS cell transfections, and the antibody material was specifically immunoprecipitated and analysed by reducing PAGE (Fig. 5). For each panel the plasmids used for transfection are as follows:

Lanes 1 and 2 - pEE6.cL.neo and pEE6.cFd.gpt (JA115) (see PCT/GB 88/00731)

Lanes 3 and 4 - pEE6.cL.neo and pEE6.cFd.δcys.Carb.

Lanes 2 and 4 - represent the results of expression in the presence of tunicamycin

The autoradiograph of the gel shows that in the absence of tunicamycin the Fd product has the molecular weight expected for a glycosylated chain (Lane 3 upper band) and that in the presence of tunicamycin the molecular weight is the same as that for the native chain whose apparent size is unaffected by the presence or absence of tunicamycin (compare the upper bands in Lanes 1, 2 and 4). These results indicate that the potential glycosylation site is used for the addition of carbohydrate. The material was able to bind specifically to the antigen for B72.3 giving equivalent signals to the native F(ab').

CHO cell lines were developed by transfection of the B72.3 chimeric light chain producing line cL 18 (see Example 1) by electroporation using supercoiled DNA of the pEE6.cFd.δcys.Carb clone. Recombinant F(ab') protein was produced by pooling initial gpt resistant transfectants and expanding the cell culture to obtain several mg of product. In parallel, several initial colonies were taken for cloning for subsequent development of stable lines.

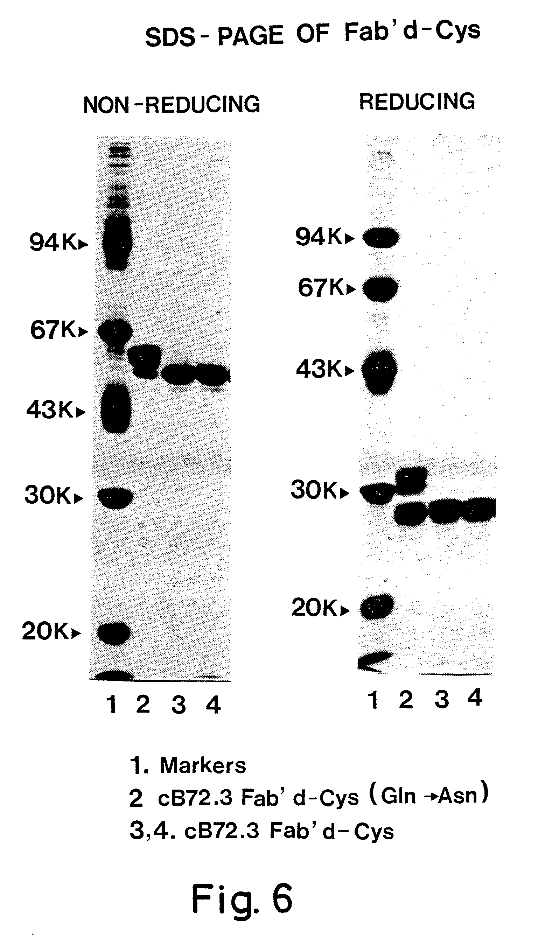

Glycosylated chimeric Fab' (cFab') was purified from CHO cell supernatant by mucin-sepharose chromatography. Bovine sub-maxillary mucin was coupled to CNBr-activated Sepharose by standard techniques. Mucin-sepharose was then prewashed with 0.1M citric acid and 2M potassium thiocyanate and then the column was equilibrated with PBS. The CHO supernatant was loaded directly onto the column, which was subsequently washed with PBS. The glycosylated cFab' was then eluted with a gradient of decreasing pH comprising 0.2M $Na_2HPO_4$ and 0.1M citric acid. Purified glycosylated cFab' was then examined by SDS-PAGE and shown to have a higher molecular weight than the non-glycosylated control (Figure 6).

Purified glycosylated cFab' and the nonglycosylated control were then iodinated with $^{125}$I by the chloramine T method [14].

Groups of four male nude mice were given subcutaneous xenografts of tumour LS174T which expresses the TAG- 72 antigen recognised by the B72.3 antibodies. Each mouse in each group was injected with 100μCi of radiolabelled cFab' or glycosylated cFab'. Groups of animals were sacrificed at intervals for the collection of tissue samples which were weighed, dissolved in 6M KOH and counted in an LKB model 1270 Rackbeta counter. Tissue uptake was calculated as the mean percentage of injected dose per gram of tissue from a group of four animals.

Results of the mouse xenograft are shown in Table 1. These results demonstrate a slower blood clearance and higher tumour uptake for the glycosylated Fab'.

Table 1

| | % injected dose per gram of tissue | | | |
|---|---|---|---|---|
| | cFab' | cFab' | glycosylated cFab' | glycosylated cFab' |
| | 1 hour | 3 hours | 1 hour | 3 hours |
| Blood | 5.26 | 2.12 | 7.62 | 3.29 |
| Liver | 2.03 | 0.94 | 2.83 | 1.23 |
| Kidney | 32.53 | 9.37 | 35.98 | 7.82 |
| Lung | 3.52 | 1.56 | 4.01 | 2.21 |
| Spleen | 1.84 | 0.82 | 2.23 | 1.15 |
| Colon | 1.87 | 0.98 | 1.94 | 1.57 |
| Muscle | 0.96 | 0.65 | 1.05 | 0.68 |
| Femur | 1.26 | 0.72 | 1.59 | 0.98 |
| Tumour | 2.99 | 2.32 | 3.27 | 3.48 |
| Biodistribution of cFab' and glyclosylated cFab' at 1 and 3 hours post-injection | | | | |

## Example 3

Construction of glycosylation site

A second example of the introduction of a glycosylation site into the CH1 domain of the B72.3 chimeric heavy chain is described below.

The site chosen was residue 204 in the CH1 domain (207 EU INDEX according to the numbering of Kabat et al (1987)). The sequence Lys Pro Ser Asn Thr Lys Val can be changed to Lys Pro Asn Asn Thr Lys Val by a single base substitution in the Serine codon from AGC → AAC to give the glycosylation motif Asn Asn Thr.

This was achieved by use of the Polymerase Chain reaction (PCR) protocol. Four oligonucleotides were prepared.

1. 5' GCT TCC ACC AAG GGC CCA TCC GTC TTC 27mer Forward primer
2. 5' CCT TGG TGT TGT TGG GCT TGT G 22mer Mutagenic primer
3. 5' CAC AAG CCC AAC AAC ACC AAG GTG 24mer Mutagenic primer
4. 5' GCC TGT GGT GCC GGG AGC CTG GTG GAA 27mer Reverse primer

Oligonucleotides 1-3 anneal within the CH1 domain; 4 anneals in the CH1-hinge intron.

All oligonucleotides were resuspended at 20μM, and 5μl were used to anneal to 50 mg of mJA108 ds DNA (see Example 2) in a final volume of 100μl of PCR buffer containing 1 unit of Taq polymerase. 30 cycles of reaction with temperatures set to 1 min 94°C; 1 min 54°C; 1 min 72°C were performed.

In the first reaction (A) oligonucleotides 1 and 2 were used and in the second reaction, B oligonucleotides 3 and 4 were used. When examined on low melting temperature agarose gels, DNA fragments of the sizes expected from the reactions were observed. These were extracted and an aliquot of A and B were mixed in a second PCR reaction to generate a fragment of 500 bp approx.

This fragment can then be cloned into the full length chimeric heavy chain as an ApaI-AccI fragment and the ability of the site to be used as a glycosylation recognition and attachment site can be verified by transfection into COS or CHO cells in association with the chimeric light chain as described in Example 1.

## Example 4

Construction of glycosylation site

9

A third example of the introduction of a glycosylation site into the CH1 domain of the B72.3 chimeric heavy chain is described below.

The site chosen was 172 (175 EU INDEX according to the numbering of Kabat et al [1987]). The sequence Val Leu Gln (172) Ser Ser Gly Leu can be changed to Val Leu Asn Ser Ser Gln Leu by a double base substitution CAG to AAC in the glutamine codon to give the glycosylation motif Asn Ser Ser.

This was achieved in the manner described in Example 2, except that oligonucleotides 2 and 3 of Example 2 were replaced by:

2. 5′ GTC CTG AGG AGT TTA GGA CAG CC 23mer Mutagenic primer

3. 5′ CCG GCT GTC CTA AAC TCC TCA GGA CTC 27mer Mutagenic primer

It will be appreciated that the present invention has been described above purely by way of illustration and that variations of detail may be made by the skilled person without departing from the scope of the invention.

REFERENCES

1. Kohler & Milstein, Nature, 265, 495-497, 1975.
2. Ehrlish, P., Collected Studies of Immunity, 2, John Willey & Sons, New York, 1906.
3. Levy & Miller, Ann. Rev. Med., 34, 107-116, 1983.
4. Schlom & Weeks, Important Advances in Oncology, 170-192, Wippincott, Philadelphia, 1985.
5. Kabat et al, Sequences of protein of Immunological Interest, Fourth Edition, U.S. Dep. of Health and Human Services, 1987.
6. Noze, M. and Wigzell, H., PNAS, 80, 6632-6636 (1983).
7. Maniatis et al, Molecular Cloning, Cold Spring Harbor, New York, 1982.
8. Primrose and Old, principles of Gene Manipulation, Blackwell, Oxford, 1980.
9. Whittle et al, Prot. Eng 1,, 6, 499-530, 1987.
10. Kieny et al, Gene, 26, 91-99, 1983.
11. Zinder, N.D., Boeke, J.D. Gene 19, 1-10 (1982)
12. Zoller, M.J., Smith, M. Nucl. Acids Res. 10 6487-6500 (1982)
13. Johnson et al, Cancer Res., 46, 850-897, 1986.
14. Kramer, W., Drusta, V., Jansen, H-W., Kramer, B., Plugfwelder, M., Frtiz, H-J., Nucl. Acids Res. 12, 9441-9456 (1984).

## Claims

1. A process for the production of an antibody having an altered number of glycosylation sites which comprises producing an expression vector containing a DNA sequence encoding a modified antibody chain having a number of glycosylation sites which is different from the number of glycosylation sites present in the natural chain from which the modified chain is derived.

2. A process according to Claim 1 including the steps of,
- transfecting or transforming host cells with the vector, and
- culturing the transformed host cells to produce an antibody molecule having an altered number of glycosylation sites.

3. A process according to Claim 2 in which the host cells are transfected or transformed with a second vector which encodes a complementary antibody heavy or light chain.

4. A process according to Claim 1 or 2, in which the vector includes a DNA sequence coding for a complementary light chain-or heavy chain- derived polypeptide.

5. A process according to Claim 3 or 4 in which the complementary antibody heavy or light chain also has an altered number of glycosylation sites.

6. A modified antibody having an altered number of glycosylation sites as compared to the number in the equivalent natural antibody.

7. A modified antibody according to Claim 6 having a reduced number of glycosylation sites.

8. A modified antibody according to Claim 6 having an increased number of glycosylation sites.

9. A modified antibody according to Claim 8, which is an Fab, an Fab′ or (Fab′)₂ Ig fragment.

10. A modified antibody according to Claim 9, in which a glycosylation site has been introduced into the CL or CH1 domain.

11. A modified antibody according to any one of Claims 6 to 10, in which the modified antibody has carbohydrate attached to its glycosylation site(s).

12. A modified antibody according to any one of Claims 6 to 11, in which an effector or reporter molecule is attached to the antibody.

13. A composition comprising a modified antibody having a number of glycosylation sites which is altered compared to the number of such sites in the equivalent natural antibody and a pharmaceutically- or diagnostically- acceptable carrier, excipient or diluent.

14. A composition according to Claim 13 for diagnostic use comprising a modified antibody according to Claim 7.

15. A composition according to Claim 13 for therapeutic use comprising a modified antibody according to Claim 8.

16. A vector comprising a DNA sequence encoding a modified antibody chain having a number of glycosylation sites which is different from the number of glycosylation sites present in the natural chain from which the modified chain is derived.

17. An expression vector according to Claim 16.

18. Host cells transformed with a vector according to Claim 16 or 17.

19. Host cells according to Claim 18 which are eucaryotic host cells.

20. The use of a modified antibody according to Claim 6 in diagnosis or therapy.

21. A modified antibody according to Claim 6 for use in diagnosis or therapy.

22. The use of an antibody according to Claim 6 for the manufacture of a medicament for use in therapy.

# Fig.1

CONSTRUCTION OF HEAVY CHAIN GENE

MUTAGENESIS Asn 291 —> GIn
Oligo 5'GTACGTGCTCTGGAACTGCTC

BglII CUT
ISOLATE Fc CONTAINING
FRAGMENT

BglII CUT
ISOLATE VECTOR
FRAGMENT

# Fig.1

### contd. CONSTRUCTION OF HEAVY CHAIN EXPRESSION CONSTRUCT

Fig. 2

# Fig. 3

## ASSAY OF ANTIGEN BINDING ACTIVITY OF RECOMBINANT ANTIBODIES

NW samples

—x—x— cL cH

—•—•— cL cH(g)

—o—o— cL cH
      +tunicamycin

x — B72·3 chimeric antibody

• — B72·3 chimeric antibody N291→φ

o — B72·3 chimeric antibody − tunicamycin treated

Samples as Fig. 2.

125-I MOUSE B72.3, CHIMER CHO+ & CHO—

LS174T XENOGRAFT, 48 HOURS

## Fig. 4

125-I MOUSE B72.3, CHIMER CHO+ & CHO—

LS174T XENOGRAFT, 48 HOURS

Fig. 5

# SDS - PAGE OF Fab'd-Cys

**NON-REDUCING**       **REDUCING**

1. Markers
2 cB72.3 Fab'd-Cys (Gln →Asn)
3,4. cB72.3 Fab'd-Cys

# Fig. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | CLINICAL CHEMISTRY, vol. 34, no. 9, September 1988, pages 1668-1675; S.L. MORRISON et al.: "Production and characterization of genetically engineered antibody molecules" * Page 1670, right-hand column * | 1-7,16-19 | C 12 N 15/13 <br> C 12 P 21/08 <br> C 12 N 5/10 <br> G 01 N 33/577 |
| E | EP-A-0 359 096 (THE TRUSTEES OF COLUMBIA UNIVERSITY IN THE CITY OF NEW YORK)(21-03-1990) * The whole document, especially page 12, last two lines * | 1-22 | |
| A | WO-A-8 807 089 (MEDICAL RESEARCH COUNCIL)(22-09-1988) * The whole document, especially page 13, lines 13-16 * | 1-7,16-19 | |
| A | WO-A-8 901 974 (CELLTECH LTD)(09-03-1989) * The whole document * | 1-7,16-19 | **TECHNICAL FIELDS SEARCHED (Int. Cl.5)** |
| P,X | THE JOURNAL OF IMMUNOLOGY, vol. 143, no. 8, 15th October 1989, pages 2595-2601, The American Association of Immunologists, US; M.-H. TAO et al.: "Studies of aglycosylated chimeric mouse-human IgG" * The whole article * | 1-7,16-19 | C 12 N <br> C 12 P |

The present search report has been drawn up for all claims

| Place of search | Date of completion of the search | Examiner |
|---|---|---|
| THE HAGUE | 21-06-1990 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P0401)